# EUROPÄISCHE PATENTSCHRIFT

(11) **EP 0 399 438 B1**
(45) Veröffentlichungstag und Bekanntmachung des Hinweises auf die Patenterteilung: **28.02.1996**
(21) Anmeldenummer: 90109611.5
(22) Anmeldetag: 21.05.1990
(51) Int. Cl.: C12Q 1/68, C07H 21/00, G01N 33/58

(54) **Verfahren zur nichtradioaktiven Messung der Nucleinsäuresynthese in eukaryontischen Zellen**
Method for the non-radioactive determination of nucleic acid synthesis in eucaryotic cells
Procédé pour la détermination de la synthèse des acides nucléiques dans les cellules eucaryotes par marquage non-radioactif

(30) Priorität: 22.05.1989 DE 3916595
(43) Veröffentlichungstag der Anmeldung: 28.11.1990
(73) Patentinhaber: BOEHRINGER MANNHEIM GMBH, D-68298 Mannheim (DE)
(72) Erfinder: Hinzpeter, Matthias, Dr., D-8000 München 21 (DE); Von der Eltz, Herbert, Dr., D-8120 Weilheim (DE)
(74) Vertreter: Huber, Bernhard, Dipl.-Chem.

(56) Entgegenhaltungen:
- EP-A- 0 063 879
- EP-A- 0 173 251
- WO-A-89/02733
- US-A- 4 210 746
- US-A- 4 585 736
- JOURNAL OF IMMUNOLOGICAL METHODS, Band 82, 1985, New York, NY (US); T. PORSTMANN et al., Seiten 169-179/
- PROCEEDINGS OF THE NATL. ACADEMY OF SCIENCES USA, Band 84, November 1987, Washington, DC (US); P.L. FELGNER et al., Seiten 7413-7417/
- EXPERIMENTAL CELL RESEARCH, Band 120, Nr. 2, 1979; Seiten 421-425/

## Beschreibung

Die Erfindung betrifft ein Verfahren zur Bestimmung der Nucleinsäure-Syntheserate eukaryontischer Zellen.

Zur Bestimmung der Nucleinsäure-Syntheserate und damit der Proliferation eukaryontischer Zellen werden bisher vorwiegend radioaktiv (³H, ³P, ¹⁴C etc.) markierte Nukleoside, welche von den Zellen in vivo aufgenommen, phosphoryliert und als Nucleinsäure-Bausteine in die zelluläre Nucleinsäure eingebaut werden, verwendet (B. Helpap und W. Maurer, Naturwissenschaften 54 (1967), 520). Die in die zelluläre Nucleinsäure eingebaute Radioaktivität kann dann mit Hilfe eines Zählers oder anderer bekannter Techniken bestimmt werden.

Es besteht eine allgemeine Tendenz, die Handhabung von radioaktiven Materialien durch die Verwendung nicht radioaktiver Markierungen zu ersetzen. Die Gründe hierfür liegen u.a. in den bekannten Gefahren im Umgang mit radioaktiven Stoffen. Jedoch sind bekannte, nicht radioaktive Markierungen, wie z.B. Fluoreszenzfarbstoffe oder Markierungsenzyme im allgemeinen Moleküle von beträchtlicher Größe und können nicht ohne weiteres in lebende Zellen eingebracht werden. Es wurde daher von H.G. Gratzner et al., Exp. Cell Res. 95 (1975), 88; und H.G. Gratzner et al., J. Histochem. Cytochem. 24, (1976), 34, vorgeschlagen, anstelle radioaktiv markierter Nukleoside das Nukleosid 5-Brom-2-desoxyuridin in Zellen einzubringen (derartige Nucleoside werden von der Zelle ohne Hilfsmittel aufgenommen, ebenso wie die radioaktiv markierten Nucleoside). Der Einbau dieses Nukleosids wurde dann mittels eines dagegen gerichteten spezifischen Antikörpers, der seinerseits mit einer Markierung versehen ist, nachgewiesen. Es wurden auch bereits Arbeiten veröffentlicht, die die Quantifizierung des Einbaus von 5-Brom-2-desoxyuridin in die zelluläre DNA mittels eines Enzymimmunoassays beschreiben (T. Porstmann et al., J. Immunol. Methods 83 (1985), 169-179; F. Martinon et al., Lab. 23 (1987), 153-159). Um das in die zelluläre DNA eingebaute 5-Brom-2-des-oxyuridin nachweisen zu können, muß jedoch die zelluläre DNA denaturiert werden. Eine solche drastische Behandlung der Zellen führt in einem in der Folge durchgeführten Zell-Elisa zu relativ hohem "background", wodurch die Syntheseratenbestimmung erschwert und ungenau wird. Wie jetzt gefunden wurde, führt ein Ersatz des Bromderivats durch ein mit einem Hapten markierten Nucleosid zu keinem Einbau desselben in die Nucleinsäure, so daß man die an sich ohne drastische Behandlung der Zellen durchführbare Haptenbestimmung nicht benutzen konnte.

Die EP-A-0 063 879 beschreibt Hapten-modifizierte Nucleotide, aus denen über enzymatische Polymerisation Oligo- bzw. Polynucleotide hergestellt werden, welche dann als Hybridisierungssonden in der biomedizinischen Forschung, der klinischen Diagnose und für rekombinante DNA-Technologie verwendet werden können. Die Herstellung der Oligo- oder Polynucleotide wird in vitro durchgeführt. Die US-A-4,585,736 beschreibt Halogensubstituierte Nucleosidderivate, die von Zellen in vivo aufgenommen werden können und nach Phosphorylierung als Substrat für die DNA-Polymerasen der Zelle dienen können. Der Nachweis dieser markierten Nucleoside erfolgt über eine Antikörperreaktion, bei der die neu synthetisierte DNA aber denaturiert werden muß, damit die antigene Determinante für den Antikörper zugänglich wird. Hierzu sind drastische Reaktionsbedingungen nötig, die zu einem relativ hohen "background" bei der Bestimmung der DNA-Syntheserate führen. Die WO-A-8902733 beschreibt die Verabreichung von Nucleosidanaloga, die antiviral wirksam sind. Diese Nucleosidanaloga werden für die Verabreichung in Form der 5'-Monophosphate in Liposomen verpackt eingesetzt. Dies bietet zwar im Hinblick auf Nebenwirkungen bei der Verabreichung (Toxizität) Vorteile, bietet jedoch keine Hinweise auf die Lösung der erfindungsgemäßen Aufgabe. In Experimental Cell Research 120, 2 (1979), 421-425 ist eine Methode zur Permeabilisierung von Zellen beschrieben, wodurch Zellen für Substanzen des Kulturmediums aufnahmefähig gemacht werden. Dies trifft jedoch nur für kleine Moleküle zu, und die Bestimmung der notwendigen Menge des Permeabilisierungsmittels Lysolecithin ist aufwendig, wobei nur ein enger Konzentrationsbereich eine Permeabilisierung und den Erhalt der Lebensfähigkeit der Zellen gewährleistet. US-A-4 210 746 beschreibt die Einbringung von Oligonucleotiden in Zellen mittels Liposomen. Es ist jedoch kein Hinweis zu entnehmen, wie Hapten-markierte Nucleotide so von Zellen aufgenommen werden könnten, daß unter Einbau dieser Nucleotide eine normale Nucleinsäuresynthese stattfinden kann. Proc. Natl. Acad. Sci. USA 84 (1987), 7413-7414 beschreibt ganz allgemein Liposomen und daß sie zur Aufnahme und Transfektion von DNA geeignet sind. Einen Hinweis auf die Einbringung von nicht-radioaktiv markierten Nucleotiden in Zellen und deren Einbau in die zelluläre DNA, wodurch die Syntheserate der Nucleinsäuren in der Zelle bestimmt werden könnte, ist auch hierin nicht beschrieben.

Aufgabe der vorliegenden Erfindung ist es daher, ein Verfahren zu schaffen, welches die Nucleinsäure-Syntheserate eukaryontischer Zellen einfach und genau nachzuweisen gestattet, wobei jedoch die Verwendung radioaktiver Verbindungen und eine Denaturierung der Zellen vermieden werden soll.

Diese Aufgabe wird erfindungsgemäß gelöst durch ein Verfahren zur Bestimmung der Nucleinsäure-Syntheserate eukaryontischer Zellen in vitro oder in Zellkultur, welches dadurch gekennzeichnet ist, daß man unter Zusatz von Liposomen ein mit einem Hapten derivatisiertes oder nicht-radioaktiv markiertes Nukleotid in die Zellen einbringt und die Nucleinsäure-Syntheserate anhand des Einbaus des Nukleotids über dessen Markierung oder Derivatisierung feststellt.

Die Erfindung beruht auf der überraschenden Feststellung, daß bei Anwendung von markierten Nucleotiden, in Gegenwart von Liposomen, im Gegensatz zur Verwendung von markierten Nucleosiden, der angestrebte Einbau in die Nucleinsäuren stattfindet und als Maß der Nucleinsäure-Syntheserate dienen kann.

Durch das erfindungsgemäße Verfahren wird es ermöglicht, ein Nucleotid-Molekül in die neu-synthetisierte Nucleinsäure (DNA/RNA) in Zellen einzubauen und dieses in der Folge aufgrund seiner Markierung zu bestimmen. Die Markierung kann vorzugsweise über einen Fluoreszenz-Farbstoff, oder durch eine Derivatisierung, welche wiederum nachweisbar ist, erfolgen (s.o.). Es kann daher anhand der Einbaurate des markierten bzw. derivatisierten Nukleotids auf die Nucleinsäure-Syntheserate in den Zellen geschlossen werden. Als Nucleotide können natürlich vorkommende oder andere Nucleotide der Ribo-, Desoxy- und Didesoxynucleotidreihe verwendet werden.

Als Markierungen sind erfindungsgemäß alle nicht radioaktiven Markierungen geeignet.

Bei der bevorzugten Verwendung eines mit einem Hapten markierten Nukleotids wird bevorzugt dessen Bestimmung über einen seinerseits markierten Anti-Hapten-Antikörper durchgeführt. Als Haptene sind alle solche geeignet, welche nicht durch übermäßige Größe den Einbau des Nukleotids in die Nucleinsäure bzw. die Syntheserate der Nucleinsäure behindern und durch einen spezifischen Anti-Hapten-Antikörper nachgewiesen werden können. Als Hapten sind bevorzugt Moleküle mit einem Molkulargewicht von 300-1200, wie Steroide und steroid-ähnliche Verbindungen (wie z.B Cortisol, Oestriol, Oestradiol, Theophyllin Testosterol, Gallensäuren, Progesteron, Aldosteron,

Digoxin, Digoxigenin, Scillarenin, Bufatalin, Ecdyson und Tomatidin); kurzkettige Peptide (wie z.B. Antipressin, Oxytocin und Bradykinin); Fluoreszenzfarbstoffe (wie z.B. Fluorescein und seine Derivate, Resorufin, Rhodamine etc.); T₃, T₄, Biotin und seine Derivate; Aflatoxin; Atrazin; Pflanzenhormone (wie z.B. Gibberilline); Alkaloide (wie z.B. Reserpin und Ajamalicin); Phenobarbitale; Vitamine (wie z.B. B 12 und seine Derivate). Erfindungsgemäß werden als Haptene bevorzugt Digoxigenin, Biotin oder Fluorescein verwendet. Als Anti-Hapten-Antikörper können monoklonale oder polyklonale Antikörper oder deren Fragmente und Derivate verwendet werden. Derartige Antikörper sind dem Fachmann bekannt.

Anstelle eines Anti-Hapten-Antikörpers kann für den Haptennachweis auch ein anderer Partner eines das Hapten als Bindungspartner umfassenden Bindungssystems verwendet werden, der selbst wiederum eine nachweisbare Markierung tragen kann. Als Partner in einem solchen Bindungssystem sind beispielsweise bei Biotin als Hapten Streptavidin oder Avidin geeignet, jedoch auch andere Moleküle vergleichbarer Größe, welche gemeinsam ein Bindungspaar bilden.

Erfindungsgemäß bevorzugt verwendete Nukleotide sind an 5-Position markierte oder derivatisierte Pyrimidin-Nucleotide, an 8-Position markierte oder derivatisierte Purin-Nucleotide oder an 7-Position markierte oder derivatisierte 7-Desazapurin-Nucleotide, jeweils mit Ribose, Desoxyribose oder Didesoxyribose als Zuckerrest, besonders bevorzugt 5-Digoxigenin-2-desoxyuridintriphosphat, welches mit einem gegen Digoxigenin gerichteten hochaffinen, monoklonalen Antikörper nachgewiesen wird. Besonders bevorzugt wird ein Fab-Fragment eines polyklonalen Antikörpers eingesetzt.

Sowohl zur nicht-radioaktiven Markierung des Nukleotids, als auch des Anti-Hapten-Antikörpers oder des markierten Partners eines Bindungssystems wird bevorzugt ein Fluoreszenz-Farbstoff verwendet. Geeignete Fluoreszenz-Farbstoffe sind dem Fachmann bekannt, erfindungsgemäß wird besonders bevorzugt Resorufin, Rhodamin, Cumarin oder Fluorescein verwendet. In einer anderen bevorzugten Ausführungsform verwendet man als Markierung des Nukleotids, des Anti- Hapten-Antikörpers oder des anderen Partners des Bindungssystems eine Enzymmarkierung und führt die Bestimmung der Einbaurate über die spezifische Substratspaltung des Markierungsenzyms durch. Hierbei kann von der enzymatischen Aktivität auf die Einbaurate des modifizierten Nukleotids und damit auf die Nucleinsäure-Syntheserate rückgeschlossen werden. Als Markierungsenzyme sind wiederum dem Fachmann bekannte Enzyme geeignet. Vorzugsweise wird β-Galactosidase, alkalische Phosphatase oder Peroxidase verwendet. Besonders bevorzugt wird die Enzymmarkierung als Markierung des Anti-Hapten-Antikörpers oder des anderen Partner des Bindungssystems verwendet.

Als Liposomen eignen sich im Rahmen der Erfindung allgemein zellmembrangängige, natürliche und synthetische Liposomen, wie sie dem Fachmann bekannt sind. Besonders geeignet sind kationische Liposomen, wie sie in der in der DE-A-01 72 007 angegebenen allgemeinen Formel I genannt sind. Weitere geeignete kationische Lipide sind in den Literaturstellen JACS 99 (1977), 3860 und Biophysical Chemistry 10 (1979), 261 beschrieben.

Es ist bevorzugt, Liposomen nach DE-A-01 72 007, in Kombination mit Phospholipiden, wie z.B. Dioleylphosphatidylethanolamin (PtdEtn) oder Dioleylphosphatidylcholin, zu verwenden. Vorzugsweise wird dem Lipid ein Phospholipid in einer Menge von bis zu 75 %, besonders bevorzugt in einer Menge von maximal 50 %, zugesetzt. Ganz besonders bevorzugt ist es jedoch, phosphatfreie Lipide zu verwenden.

Liposomen werden beispielsweise dadurch hergestellt, daß die Lipide in Wasser suspendiert werden und bis zum Erhalt einer klaren Lösung, beispielsweise durch Ultraschall oder Verpressung durch kleine Düsen behandelt werden. Nähere Angaben zu erfindungsgemäß geeigneten Liposomen finden sich z.B. in Gregory Gregoriadis (1984) Preparation of Liposomes, Liposome Technology, Vol. 1, C.R.C. Press, Boca Raton, Florida.

Bevorzugt werden aus N-[1-(1,2-dioleyloxy)propyl]-N,N,N-trimethylammoniumchlorid gebildete Liposomen verwendet. Die Verwendung von diesen DOTMA-Liposomen wurde von P. L. Felgner et al., Proc. Natl. Acad. Sci. USA 84, (1987) 7413-7417 für die Transfektion von DNA in eukaryontischen Zellen beschrieben.

Zur Einbringung der Liposomen in die Zellen wird vorzugsweise ein serumhaltiges Wachstumsmedium verwendet.

Die Bestimmung der Syntheserate über die Markierung des Nukleotids, Anti-Hapten-Antikörpers oder anderen Partners des Bindungssystems wird bei Verwendung eines Fluoreszenz-Farbstoffs bevorzugt über die Immunfluoreszenz der proliferierenden Zellen vorgenommen oder die Fluoreszenz wird direkt gemessen.

In anderen bevorzugten Ausführungsformen der Erfindung wird zur Bestimmung eine immuncytochemische Untersuchung der proliferierenden Zellen angewandt, oder aber ein Zell-ELISA (enzyme linked immunosorbent assay) durchgeführt. Hierdurch wird es auf leichte und schnell durchführbare Weise ermöglicht, eine sehr genaue Bestimmung der Nucleinsäure-Syntheserate der Zellen durchzuführen, wobei keine Verfälschung der Ergebnisse durch hohen Background aufgrund von Denaturierung der zellulären Nucleinsäure gegeben ist.
In eukaryontische Zellen können mittels Liposomen aus kationischen Detergentien auch Kombinationen von nicht radioaktiv markierten Nukleotiden mit anderen nicht markierten Substanzen, wie z.B. Inositolphoshate, DNA und RNA, Therapeutica, Hormone, wie z. B. Östrogene, eingebracht werden, mit dem Ziel, die für die eingebrachten nicht markierten Substanzen spezifische Nukleinsäuresythese zu messen.
Mit einer Kombination von markiertem Nukleotid mit z.B. unmarkiertem Inositoltriphoshat kann nach den beschriebenen Verfahren die Nukleinsäuresynthese gemessen werden, die durch das Inositoltriphosphat induziert wird.
Mit einer Kombination von markiertem Desoxyribonukleotid mit unmarkierter DNA, z.B. Plasmid DNA, kann die replikative Nukleinsäuresynthese dieses Plasmides nachgewiesen werden, indem z.B. mittels Southern blot das unmarkierte Plasmid auf eine Membran transferiert wird und dann gegen die markierte neu synthetisierte (replizierte) Plasmid DNA hybridisiert wird. Mit einer Kombination von markiertem Ribonukleotid mit unmarkierter mRNA, kann die transkriptive Nukleinsäuresythese nachgewiesen werden, indem z.B. mittels Northern blot die unmarkierte mRNA auf eine Membran transferiert und dann gegen die markierte, neu synthetisierte (transkribierte) mRNA hybridisiert wird.
Mit einer Kombination von markiertem Ribonukleotid mit unmarkierter DNA, z.B. Plasmid DNA kann die transkriptive Nukleinsäuresynthese dieses Plasmides nachgewiesen werden, indem z.B. mittels Southern blot das unmarkierte Plasmid auf eine Membran transferiert wird und dann gegen die markierte neu synthetisierte (transkribierte) mRNA hybridisiert wird.
Mit einer Kombination von markiertem Desoxyribonukleotid mit unmarkierter RNA, kann die revers transkriptive Nukleinsäuresynthese, z.B. in HIV infizierten Zellen, nachgewiesen werden, indem z.B. mittels Northern blot die unmarkierte RNA auf eine Membran transferiert wird und dann gegen die markierte neu synthetisierte (revers transkribierte) DNA hybridisiert wird. In gleicher Weise kann eine Kombination von markiertem Desoxyribonucleotid mit Didesoxynucleotiden verwendet werde. Die folgenden Beispiele erläutern die Erfindung.

### Beispiel 1

### Herstellung der Liposomen

Eine Lösung von 20 mg N-[1-(2,3-diolyloxy)propyl]-N,N,N-trimethylamoniumchlorid (DOTMA) in 1 ml Chloroform wird bis zur Trockne unter Stickstoff eingedampft und über Nacht unter Vakuum getrocknet. Der Rückstand wird in 2 ml H₂O bidest. resuspendiert und mit Ultraschall bei 4°C solange beschallt bis die Lösung klar ist. Die Lösung wird mit Hepes buffered saline (HBS) auf eine Konzentration von 1 mg/ ml verdünnt und durch einen 0.2 µm Filter sterilfiltriert.

### Beispiel 2

### Transfer von Digoxigenin-11-dUTP in adhärente Zellen

**a) Fluoreszenzdetektion**
   Zu 20 µl einer Lösung der nach Beispiel 1 hergestellten Liposomen (1 mg/ml) werden 10 µl Digoxigenin-11-dUTP (Herstellung nach DE 38 13 278; 10 mg/ml in HBS) gegeben und gemischt. Diese Mischung wird zu adhärent auf integrated chamber slides (Miles) wachsenden Zellen (CHO-Zellen, ATCC: CCL 61) zugegeben (Medium: Zellkulturmedium DMEM, Boehringer Mannheim GmbH 500 µl mit 10 % foetalem Kälberserum) und 2 h im Inkubator (37°C, 5% CO₂) inkubiert.
   Der Überstand wird abgesaugt und die Zellen 3x mit phosphate buffered saline, NaCl, 137 mmol/l; KCl, 2.7 mmol/l; Na₂HPO₄, 8 mmol/l; KH₂PO₄, 1.5 mmol/l; pH 7.5; (PBS) (37°C), bei Raumtemperatur jeweils 2 min gewaschen. Nach dem letzten Absaugen werden die Zellen mit 100% Methanol, -20°C, 10 min bei -20°C fixiert. Das Methanol wird abgesaugt und die Zellen wie oben beschrieben 3x mit PBS gewaschen. Nach dem letzten Absaugen wird 30 min mit reinem FKS bei 37°C inkubiert. Anschließend wird das FKS abgesaugt und wie oben beschrieben 3x mit PBS gewaschen. Nach dem letzten Absaugen werden die Fab-Fragmente eines polyklonalen Antikörpers, gerichtet gegen Digoxigenin, die mit 5(6)-Carboxy-X-Rhodamin (Boehringer Ma.) konjugiert sind, zugegeben und 1 h bei 37°C inkubiert. Dann wird wie oben beschrieben 3x mit PBS gewaschen. Nach dem letzen Absaugen werden die feuchten Objektträger in mounting medium, wie z.B. Mowiol^{R} (Fa. Höchst), eingebettet und 1 h im Dunkeln bei Raumtemperatur getrocknet. Im Fluoreszenz-Mikroskop ist bei Grünanregung (572 nm) eine rote Fluoreszenz der Zellkerne sichtbar.
**b) Enzymdetektion**Zu 10 µl einer Lösung der nach Beispiel 1 hergestellten
   Liposomen (1mg/ ml) werden 5 µl Digoxigenin-11-dUTP (10 mg/ml in HBS) gegeben und gemischt. Diese Mischung wird zu adhärent in Mikrotiterplatten (Fa. Nunc) wachsenden Zellen (CHO-Zellen ATCC: CCL 61) zugegeben(Medium: Zellkulturmedium, DMEM, Boehringer Mannheim GmbH, 500 µl mit 10% foetalem Kälberserum) und 2 h im Inkubator
   (37°C, 5% CO₂) inkubiert. Der Überstand wird abgesaugt und die Zellen 3x mit Tris buffered saline, Tris-HCl, 50 mmol/l; NaCl, 150 mmol/l; pH 7.5; (TBS), (37°C) bei Raumtemperatur jeweils 2 min gewaschen. Nach dem letzten Absaugen werden die Zellen mit 100% Methanol, -20°C, 10 min bei -20°C fixiert. Das Methanol wird abgesaugt und die Zellen wie oben beschrieben 3x mit TBS gewaschen. Nach dem letzten Absaugen wird 30 min mit reinem FKS bei 37°C inkubiert. Anschließend wird das FKS abgesaugt und wie oben beschrieben 3x mit TBS gewaschen. Nach dem letzten Absaugen werden die Fab-Fragmente eines Polyklonalen Antikörpers, gerichtet gegen Digoxigenin, die mit Alkalischer Phosphatase (AP) konjugiert sind, zugegeben und 1 h bei 37°C inkubiert. Dann wird wie oben beschrieben 3x mit TBS gewaschen. Nach dem letzen Absaugen wird die gebundene AP mit dem Substrat p-Nitrophenylphosphat im Mikrotiterplattenreader (Fa. SLT), 405 nm, detektiert.

### Beispiel 3

### Transfer von Digoxigenin-11-UTP in adhärente Zellen

**a)**
   Es wird wie in Beispiel 2 a) verfahren, wobei anstelle von Digoxigenin-11-dUTP Digoxigenin-11-UTP (Herstellung analog DE 38 13 278) eingesetzt wird.
   Im Fluoreszenz-Mikroskop ist bei Grünanregung (572 nm) eine rote Fluoreszenz der gesamten Zelle sichtbar.
**b)**
   Es wird wie in Beispiel 2 b) verfahren, wobei anstelle von Digoxigenin-11-dUTP Digoxigenin-11-UTP eingesetzt wird.

### Beispiel 4

### Transfer von Digoxigenin markierter DNA in adhärente Zellen

**a)**
   Es wird wie in Beispiel 2 a) verfahren, wobei anstelle von Digoxigenin-11-dUTP 10 µl Digoxigenin markierte DNA (100 ug/ml, * pBR 328) eingesetzt wird (Herstellung analog DE 38 13 278). Im Fluoreszenz-Mikroskop ist bei Grünanregung (572 nm) eine rote Fluoreszenz der gesamten Zelle sichtbar.
**b)**
   Es wird wie in Beispiel 2 b) verfahren, wobei anstelle von Digoxigenin-11-dUTP 10 µl Digoxigenin markierte DNA (100 ug/ml, pBR 328) eingesetzt wird (Herstellung analog DE 38 13 278).

* (ug/ml = µg/ml)

### Beispiel 5

### Transfer von Digoxigenin markierter RNA in adhärente Zellen

**a)**
   Es wird wie in Beispiel 2 a) verfahren, wobei anstelle von Digoxigenin-11-dUTP 10 µl Digoxigenin markierte RNA (100 ug/ml), wie z.B. das Transkript des Neomycin Gen eingesetzt wird (Herstellung analog DE 38 13 278).
   Im Fluoreszenz-Mikroskop ist bei Grünanregung (572 nm) eine rote Fluoreszenz der gesamten Zelle sichtbar.
**b)**
   Es wird wie in Beispiel 2 b) verfahren, wobei anstelle von Digoxigenin-11-dUTP 10 µl Digoxigenin markierte RNA 100 ug/ml), wie z.B. das Transkript des Neomycin Gen eingesetzt wird (Herstellung analog DE 38 13 278).

### Beispiel 6

### Herstellung von Fluorescein-dUTP

**a) 5(6)-Carboxyfluorescein-ε-amidocaproyl-[5-(amidoallyl)-2′-desoxy-uridin-5′-triphosphat]-tetralithiumsalz (Fluorescein-dUTP)**
586 mg 5(6)-Carboxyfluorescein-ε-amidocapronsäure-N-hydroxysuccinimidester (1 mmol) (hergestellt in einer zweistufigen Synthese durch Umsetzung von 5(6)-Carboxyfluorescein-N-hydroxysuccinimidester (FLUOS, Boehringer Mannheim 1055089) mit 6-Aminocapronsäure und nachfolgender Herstellung des N-Hydroxysuccinimidesters) werden in 20 ml Dimethylformamid (DMF) gelöst und zu einer Lösung von 547 mg 5-Aminoallyl-2′-desoxy-uridin-5′-triphosphat-tetralithiumsalz (1 mmol) in 20 ml 0,1 mol/l Natriumboratpuffer, pH 8,5 gegeben. Das Gemisch wird über Nacht (ca. 15 h) bei Raumtemperatur gerührt, wonach papierelektrophoretisch (0,05 mol/l Citratpuffer, pH 5,0) ein fast quantitativer Umsatz zum gewünschten Produkt beobachtet wird.
Das Reaktionsgemisch wird im Vakuum eingedampft, der Rückstand in 250 ml dest. Wasser aufgenommen und zwecks Ionenaustauscher-Chromatographie auf eine DEAE-Sephadex A-25-Säule (2x40 cm) in der Chlorid-Form gegeben. Man eluiert mit einem linearen Gradienten von Wasser auf 0,3 mol/l-LiCl, vereinigt die das Produkt enthaltenden Fraktionen und konzentriert diese im Vakuum auf ein Gesamtvolumen von ca. 50 ml. Dieses Konzentrat wird unter kräftigem Rühren in 500 ml eines Gemisches von Aceton/Ethanol 3:1 getropft, wobei das Nucleotid ausfällt.
Das Präzipitat wird abzentrifugiert und mehrfach mit Aceton/Ethanol 3:1 bis zur Chlorid-Freiheit gewaschen. Nach Trocknung im Vakuum über P₂O₅/KOH werden 453 mg (45% der Theorie) der Titelverbindung in Form eines gelben Pulvers erhalten.

| Elementaranalyse für C₃₉H₃₇O₂₄N₄P₃Li₄ : | |
|---|---|
| C_{ber} 43,9% | C_{gef} 43,5% |
| H_{ber} 3,5% | H_{gef} 3,65% |
| N_{ber} 5,25% | N_{gef} 5,0% |
| P_{ber} 8,7% | P_{gef} 8,9% |

³¹P-NMR (D₂O): δ = -5,2 (d, P_{γ}), -10,3 (d, P_{α}), -21,3 (t, P_{β})
**b)** **5(6)-Carboxyfluorescein-ε-amidocaproyl-[5-tamidoallyl)-uridin-5'-triphosphat]-tetralithiumsalz**
Analog Beispiel 6a) werden 586 mg 5(6)-Carboxyfluorescein-ε-amidocapronsäure-N-hydroxysuccinimidester (1 mmol) mit 563 mg 5-Aminoallyl-uridin-5'-triphosphattetralithiumsalz (1 mmol) in DMF/0,1 mol/l Natriumboratpuffer pH 8,5 umgesetzt.
Die Aufarbeitung des Reaktionsgemisches erfolgt ebenfalls in der in Beispiel 6a) angegebenen Weise, wonach 520 mg (50% der Theorie) der gewünschten Verbindung als gelboranges amorphes Pulver erhalten werden.

| Elementaranalyse für C₃₉H₃₇O₂₄N₄P₃Li₄ : | |
|---|---|
| C_{ber} 43,3% | C_{gef} 42,9% |
| H_{ber} 3,4% | H_{gef} 3,5% |
| N_{ber} 5,2% | N_{gef} 5,1% |
| P_{ber} 8,6% | P_{gef} 8,75% |

³¹P-NMR-Spektrum identisch mit dem des entsprechenden 2'-Desoxy-Derivates.

### Beispiel 7

### Transfer von Fluorescein-dUTP in adhärente Zellen

**a)**
   Zu 20 µl einer Lösung der nach Beispiel 1 hergestellten Liposomen (1 mg/ml) werden 10 µl Fluorescein-dUTP (Beispiel 6a) gegeben und gemischt. Diese Mischung wird zu: adhärent auf integrated chamber slides (Miles) wachsenden Zellen (CHO-Zellen, ATCC: CCL 61) zugegeben (Medium: Zellkulturmedium DMEM, Boehringer Mannheim GmbH, 500 µl mit 10% foetalem Kälberserum)
   und 2 h im Inkubator (37°C, 5% CO₂) inkubiert. Der Überstand wird abgesaugt und die Zellen 3x mit (PBS), (37°C) bei Raumtemperatur jeweils 2 min gewaschen. Nach dem letzten Absaugen werden die Zellen mit 100% Methanol, -20°C, 10 min bei -20°C fixiert. Das Methanol wird abgesaugt und die Zellen wie oben beschrieben 3x mit PBS gewaschen. Nach dem letzen Absaugen werden die feuchten Objektträger in mounting medium, wie z.B. Mowiol^{R} (Fa. Höchst) eingebettet und 1 h im Dunkeln bei Raumtemperatur getrocknet. Im Fluoreszenz-Mikroskop ist bei Blauanregung (494 nm) eine grüne Fluoreszenz der Zellkerne sichtbar.
   Die Fluoreszenz-markierten Zellen können auch im FACS (Fluorescence Activated Cell Sorter) analysiert werden. Für diese Anwendung kann auf die Methanol Fixierung verzichtet werden.
**b)**
   Zu 20 µl einer Lösung der nach Beispiel 1 hergestellten Liposomen (1 mg/ml) werden 10 µl Fluorescein-dUTP gegeben und gemischt. Diese Mischung wird zu adhärent auf Mikrotiterplatten (Fa. Nunc) wachsenden Zellen (CHO-Zellen) (Medium: Zellkulturmedium DMEM, Boehringer Mannheim GmbH, 500 µl mit 10 % Foetalem Kälberserum) zugegeben
   und 2 h im Inkubator (37°C, 5% CO₂) inkubiert. Der Überstand wird abgesaugt und die Zellen 3x mit (PBS), (37°C) bei Raumtemperatur jeweils 2 min gewaschen. Nach dem letzten Absaugen werden die Zellen mit 100% Methanol, -20°C, 10 min bei -20°C fixiert. Das Methanol wird abgesaugt und die Zellen wie oben beschrieben 3x mit PBS gewaschen. Die Detektion erfolgt im Fluoreszenzphotometer (Fa. Flow).
   Die Fluoreszenz-markierten Zellen können auch nach bekannten Methoden im FACS (Fluorescence Activated Cell Sorter) analysiert werden. Für diese Anwendung kann auf die Methanol Fixierung verzichtet werden.

### Beispiel 8

### Transfer von Digoxigenin-11-dUTP in Suspensionszellen

**a) Fluoreszenzdetektion**
   Zu 20 pl einer Lösung der nach Beispiel 1 hergestellten Liposomen (1mg/ ml) werden 10 pl Digoxigenin-11-dUTP (10 mg/ml in HBS) gegeben und gemischt. Diese Mischung wird zuⁱⁿ integrated chamber slides (Miles) in Suspension wachsenden Zellen (NS-1, ATCC: TIB 18) (Medium: Zellkulturmedium DMEM, Boehringer Mannheim GmbH, 500 µl mit 10 % Foetalem Kälberserum) zugegeben und 2 h im Inkubator (37°C, 5% CO₂) inkubiert. Vor dem Absaugen des Überstandes werden die Zellen auf den Boden des Objektträgers zentrifugiert (Zytospinzentrifuge, Fa. Shandon, 30 g) und dann der Überstand vorsichtig abgesaugt. Die Zellen werden 3x mit PBS (37°C), bei Raumtemperatur jeweils 2 min gewaschen. Vor dem Absaugen des Waschpuffers müssen die Zellen, wie oben beschrieben, jeweils auf den Boden des Objektträgers zentrifugiert werden. Nach dem letzten Absaugen werden die Zellen auf den Objekträger bei 37°C, 1 h, angetrocknet und dann mit 100% Methanol, -20°C, 10 min bei -20°C fixiert. Das Methanol wird abgesaugt und die Zellen wie oben beschrieben 3x mit PBS gewaschen. Nach dem letzten Absaugen wird 30 min mit reinem FKS bei 37°C inkubiert. Anschließend wird das FKS abgesaugt und die Zellen wie oben beschrieben 3x mit PBS gewaschen. Nach dem letzten Absaugen werden die Fab-Fragmente eines Polyklonalen Antikörpers, gerichtet gegen Digoxigenin, die mit 5(6)-Carboxy-X-Rhodamin konjugiert sind, zugegeben und 1 h bei 37°C inkubiert. Dann wird wie oben beschrieben 3x mit PBS gewaschen. Nach dem letzen Absaugen werden die feuchten Objektträger in mounting medium, wie z.B. Mowiol (Fa. Höchst) eingebettet und 1 h im Dunkeln bei Raumtemperatur getrocknet. Im Fluoreszenz-Mikroskop ist bei Grünanregung (572 nm) eine rote Fluoreszenz der Zellkerne sichtbar.
**b) Enzymdetektion**
   Zu 10 µl einer Lösung der nach Beispiel 1 hergestellten Liposomen (1mg/ ml) werden 5 µl Digoxigenin-11-dUTP (10 mg/ml in HBS) gegeben und gemischt. Diese Mischung wird zu in Mikrotiterplatten (Fa. Nunc) in Suspension wachsenden Zellen (NS-1, ATCC: TIB 18) (Medium: Zellkulturmedium DMEM, Boehringer Mannheim GmbH, 500 µl mit 10% Foetalem Kälberserum) zugegeben
   und 2 h im Inkubator (37°C, 5% CO₂) inkubiert. Vor dem Absaugen des Überstandes werden die Zellen auf den Boden der Mikrotiterplatte zentrifugiert (Zentrifuge mit Mikrotiterplatten-Einsätzen, Fa. Hettich, 200 g) und dann der Überstand vorsichtig abgesaugt. Die Zellen werden 3x mit TBS (37°C), bei Raumtemperatur jeweils 2 min gewaschen. Vor dem Absaugen des Waschpuffers müssen die Zellen, wie oben beschrieben, jeweils auf den Boden der Mikrotiterplatte zentrifugiert werden. Nach dem letzten Absaugen werden die Zellen auf dem Boden der Mikrotiterplatte bei 37°C, 1 h, angetrocknet und dann mit 100% Methanol, -20°C, 10 min bei -20°C fixiert. Das Methanol wird abgesaugt und die Zellen wie oben beschrieben 3x mit TBS gewaschen. Nach dem letzten Absaugen wird 30 min mit reinem FKS bei 37°C inkubiert. Anschließend wird das FKS abgesaugt und die Zellen wie oben beschrieben 3x mit TBS gewaschen. Nach dem letzten Absaugen werden die Fab-Fragmente eines Polyklonalen Antikörpers, gerichtet gegen Digoxigenin, die mit Alkalischer Phosphatase (AP) konjugiert sind, zugegeben und 1 h bei 37°C inkubiert. Dann wird wie oben beschrieben 3x mit TBS gewaschen. Nach dem letzen Absaugen wird die gebundene AP mit dem Substrat p-Nitrophenylphoshat im Mikrotiterplattenreader (Fa. SLT), 405 nm, detektiert.

### Beispiel 9

### Transfer von Digoxigenin-11-UTP in Suspensionszellen

**a)**
   Es wird wie in Beispiel 7 a) verfahren, wobei anstelle von Digoxigenin-11-dUTP Digoxigenin-11-UTP eingesetzt wird. Im Fluoreszenz-Mikroskop ist bei Grünanregung (572 nm) eine rote Fluoreszenz der gesamten Zelle sichtbar.
**b)**
   Es wird wie in Beispiel 7 b) verfahren, wobei anstelle von Digoxigenin-11-dUTP Digoxigenin-11-UTP eingesetzt wird.

### Beispiel 10

### Transfer von Digoxigenin markierter DNA in Suspensionszellen

**a)**
   Es wird wie in Beispiel 7 a) verfahren, wobei anstelle von Digoxigenin-11-dUTP 10 µl Digoxigenin markierte DNA (100 µg/ml, pBR 328) eingesetzt wird.
   Im Fluoreszenz-Mikroskop ist bei Grünanregung (572 nm) eine rote Fluoreszenz der gesamten Zelle sichtbar.
**b)**
   Es wird wie in Beispiel 7 b) verfahren, wobei anstelle von Digoxigenin-11-dUTP 10 µl Digoxigenin markierte DNA (100 µg/ml, pBR 328) eingesetzt wird.

### Beispiel 11

### Transfer von Digoxigenin markierter RNA in Suspensionszellen

**a)**
   Es wird wie in Beispiel 7 a) verfahren, wobei anstelle von Digoxigenin-11-dUTP 10 µl Digoxigenin markierte RNA (100 µg/ml), wie z.B. das Transkript des Neomycin Gens, eingesetzt wird.
   Im Fluoreszenz-Mikroskop ist bei Grünanregung (572 nm) eine rote Fluoreszenz der gesamten Zelle sichtbar.
**b)**
   Es wird wie in Beispiel 7 b) verfahren, wobei anstelle von Digoxigenin-11-dUTP 10 µl Digoxigenin markierte RNA (100 pg/ml), wie z.B. das Transkript des Neomycin Gens, eingesetzt wird.

### Beispiel 12

### Transfer von Fluorescein-dUTP in Suspensionszellen

**a)**
   Zu 20 µl einer Lösung der nach Beispiel 1 hergestellten Liposomen (lmg/ ml) werden 10 µl Fluorescein-dUTP (10 mg/ml in HBS) gegeben und gemischt. Diese Mischung wird zu in integrated chamber slides (Miles) in Suspension wachsenden Zellen (NS-1, ATCC: TIB 18 (Medium: Zellkulturmedium DMEM, Boehringer Mannheim GmbH, 500µl mit 10% Foetalem Kälberserum) zugegeben
   und 2 h im Inkubator (37°C, 5% CO₂) inkubiert. Vor dem Absaugen des Überstandes werden die Zellen auf den Boden des Objektträgers zentrifugiert (Zytospinzentrifuge, Fa. Shandon, 30 g) und dann der Überstand vorsichtig abgesaugt. Die Zellen werden 3x mit PBS (37°C), bei Raumtemperatur jeweils 2 min gewaschen. Vor dem Absaugen des Waschpuffers müssen die Zellen, wie oben beschrieben, jeweils auf den Boden des Objektträgers zentrifugiert werden. Nach dem letzten Absaugen werden die Zellen auf den Objekträger bei 37°C, 30 min, angetrocknet und dann mit 100% Methanol, -20°C, 10 min bei -20°C fixiert. Das Methanol wird abgesaugt und die Zellen wie oben beschrieben 3x mit PBS gewaschen. Nach dem letzen Absaugen werden die feuchten Objektträger in mounting medium, wie z.B. Mowiol (Fa. Höchst) eingebettet und 1 h im Dunkeln bei Raumtemperatur getrocknet. Im Fluoreszenz-Mikroskop ist bei Blauanregung (494 nm) eine grüne Fluoreszenz der Zellkerne sichtbar.
   Die Fluoreszenz-markierten Zellen können auch im FACS (Fluorescence Activated Cell Sorter) analysiert werden. Für diese Anwendung kann auf die Methanol Fixierung verzichtet werden.
**b)**
   Zu 10 µl einer Lösung der nach Beispiel 1 hergestellten Liposomen (lmg/ ml) werden 5 µl Fluorescein-dUTP (10 mg/ml in HBS) gegeben und gemischt. Diese Mischung wird zu: in Mikrotiterplatten (Fa. Nunc) in Suspension wachsenden Zellen (NS-1, ATCC: TIB 18) (Medium: Zellkulturmedium DMEM, Boehringer Mannheim GmbH, 500 µl mit 10% Foetalem Kälberserum) zugegeben
   und 2 h im Inkubator (37°C, 5% CO₂) inkubiert. Vor dem Absaugen des Überstandes werden die Zellen auf den Boden der Mikrotiterplatte zentrifugiert (Zentrifuge mit Mikrotiterplatten-Einsätzen, Fa. Hettich, 200 g) und dann der Überstand vorsichtig abgesaugt. Die Zellen werden 3x mit PBS (37°C), bei Raumtemperatur jeweils 2 min gewaschen. Vor dem Absaugen des Waschpuffers müssen die Zellen, wie oben beschrieben, jeweils auf den Boden der Mikrotiterplatte zentrifugiert werden. Nach dem letzten Absaugen werden die Zellen auf dem Boden der Mikrotiterplatte bei 37°C, 1 h, angetrocknet und dann mit 100% Methanol, -20°C, 10 min bei -20°C fixiert. Das Methanol wird abgesaugt und die Zellen wie oben beschrieben 3x mit PBS gewaschen. Die Detektion erfolgt im Fluoreszenzphotometer (Fa. Flow).
   Die Fluoreszenz-markierten Zellen können auch im FACS (Fluorescence Activated Cell Sorter) analysiert werden. Für diese Anwendung kann auf die Methanol Fixierung verzichtet werden.

### Beispiel 13

**Transfer von Digoxigenin-11-dUTP in adhärente Zellen analog Verfahren in PNAS 84 (1987)** 7413-7417)

20 µl einer Lösung der nach Beispiel 1 hergestellten Liposomen (1 mg/ml) werden mit HBS auf 250 µl verdünnt. 10 µl Digoxigenin-11-dUTP (10 mg/ml) werden mit HBS auf 250 µl verdünnt. Beide Lösungen werden gemischt und auf Zellen gegeben (CHO), die vorher mit HBS gewaschen wurden, um Serumreste zu entfernen und 2 h im Inkubator (37°C, 5% CO₂) inkubiert. Dann wurde analog Beispiel 2 verfahren.
Im Fluoreszenz-Mikroskop ist bei Grünanregung (572 nm) nur eine sehr schwache Fluoreszenz der Zellkerne sichtbar

### Beispiel 14

**Herstellung von Liposomen, die markiertes Nukleotid enthalten**

50 mg 1,2-Dioleyl-sn-glycero-3-phosphorylcholin wird in 5 ml Chloroform gelöst und das Lösungsmittel bei 30°C Wasserbadtemperatur am Rotationsverdampfer abgezogen. Anschließend wird 5 mg Digoxigenin markiertes dUTP, gelöst in 25 ml bidest. Wasser, zugegeben und 10 min kontinuierlich ultrabeschallt und durch einen 0.2 µm Filter sterilfiltriert.

### Beispiel 15

**Transfer von Digoxigenin-11-dUTP in Zellen mittels in Beispiel 14 hergestellter Liposomen.**

333 µl der in Bsp. 14 hergestellten Liposomen werden mit 166 µl Kulturmedium mit Serum (Zusammensetzung wie in Beispiel 2) versetzt und gemischt. Nach Absaugen des Kulturmediums werden bei Verwendung von Mikrotiterplatten 250 µl, bei Verwendung von integrated chamber slides die gesamte Mischung zu den Zellen gegeben. Nach 2h Inkubation (37°C, 5% CO₂) wird analog den anderen Beispielen verfahren.

## Patentansprüche

1. Verfahren zur Bestimmung der Nucleinsäuren-Syntheserate eukaryontischer Zellen in vitro oder in Zellkultur,
**dadurch gekennzeichnet,**
daß man unter Zusatz von Liposomen ein mit einem Hapten derivatisiertes oder nicht-radioaktiv markiertes Nukleotid in die Zellen einbringt und die Nucleinsäuren-Syntheserate anhand des Einbaus des Nukleotids über dessen Markierung oder Derivatisierung feststellt.

2. Verfahren nach Anspruch 1, **dadurch gekennzeichnet** , daß man ein Nukleotid verwendet, das mit einem Hapten derivatisiert ist, welches ein Partner eines Bindungspaares ist, und die Bestimmung über den anderen Partner durchführt, der seinerseits eine Markierung trägt.

3. Verfahren nach Anspruch 2, **dadurch gekennzeichnet,** daß man als Partner einen markierten Anti-Hapten-Antikörper verwendet.

4. Verfahren nach einem der vorhergehenden Ansprüche, **dadurch gekennzeichnet**, daß man als Markierung des Nukleotids, des Anti-Hapten-Antikörpers oder des anderen Partners des Bindungssystems einen Fluoreszenzfarbstoff oder lumineszierenden Farbstoff verwendet.

5. Verfahren nach Anspruch 2 oder 3, **dadurch gekennzeichnet**, daß man als Markierung des Anti- Hapten-Antikörpers oder des anderen Partners des Bindungssystems eine Enzymmarkierung verwendet, und die Bestimmung über spezifische Substratspaltung des Markierungsenzyms durchführt.

6. Verfahren nach einem der vorhergehenden Ansprüche, **dadurch gekennzeichnet**, daß man die Zellen während der Einbringung der Liposomen und markierten Nucleotide in Zellkulturmedium mit Serumzusatz hält.

7. Verfahren nach Anspruch 4, **dadurch gekennzeichnet**, daß man bei Verwendung eines Fluoreszenzfarbstoffs als Markierung die Bestimmung über eine Immunfluoreszenz der proliferierenden Zellen vornimmt oder die Fluoreszenz direkt mißt.

8. Verfahren nach einem der vorhergehenden Ansprüche, **dadurch gekennzeichnet**, daß man zur Bestimmung eine immuncytochemische Untersuchung von proliferierenden Zellen durchführt.

9. Verfahren nach einem der vorhergehenden Ansprüche, **dadurch gekennzeichnet**, daß man die Bestimmung über einen Zell-ELISA durchführt.

10. Verfahren nach einem der vorhergehenden Ansprüche, **dadurch gekennzeichnet**, daß man aus N-[1(2,3-dioleyloxy)propyl]-N,N,N-trimethylammoniumchlorid gebildete Liposomen verwendet.

## Claims

1. Method for the determination of the nucleic acid synthesis of eukaryotic cells in vitro or in cell culture,
**wherein**
a nucleotide which is non-radioactively labelled or derivatized with a hapten is introduced into the cells with the addition of liposomes and the rate of nucleic acid synthesis is determined on the basis of the incorporation of the nucleotide via its label or derivatization.

2. Method as claimed in claim 1,
**wherein**
a nucleotide is used which is derivatized with a hapten which is a partner of a binding pair and the determination is carried out via the other partner which itself carries a label.

3. Method as claimed in claim 2,
**wherein**
a labelled anti-hapten antibody is used as the partner.

4. Method as claimed in one of the previous claims,
**wherein**
a fluorescent dye or luminescent dye is used to label the nucleotide, the anti-hapten antibody or the other partner of the binding system.

5. Method as claimed in claim 2 or 3,
**wherein**
an enzyme label is used to label the anti-hapten antibody or the other partner of the binding system and the determination is carried out by means of the specific substrate cleavage of the enzyme label.

6. Method as claimed in one of the previous claims,
**wherein**
during the incorporation of the liposomes and labelled nucleotides the cells are kept in a cell culture medium containing a supplement of serum.

7. Method as claimed in claim 4,
**wherein**
when using a fluorecent dye as the label, the determination is carried out via an immunofluorescentce of the proliferating cells or the fluorescence is measured directly.

8. Method as claimed in one of the previous claims,
**wherein**
an immunocytochemical examination of proliferating cells is carried out for the determination.

9. Method as claimed in one of the previous claims,
**wherein**
the determination is carried out by means of a cell-ELISA.

10. Method as claimed in one of the previous claims,
**wherein**
liposomes formed from N-[1(2,3-dioleyloxy)propyl]-N,N,N-trimethylammonium chloride are used.

## Revendications

1. Procédé pour déterminer le taux de synthèse des acides nucléiques de cellules eucaryotes in vitro ou en culture de cellules, caractérisé en ce que, en ajoutant des liposomes, on introduit dans les cellules un nucléotide transformé en dérivé avec un haptène ou marqué de manière non radioactive et on détermine le taux de synthèse des acides nucléiques à l'aide de l'incorporation du nucléotide par le biais de son marqueur ou de sa transformation en dérivé.

2. Procédé selon la revendication 1, caractérisé en ce que l'on utilise un nucléotide qui est transformé en dérivé avec un haptène qui est un partenaire d'une paire de liaison et on réalise la détermination par le biais de l'autre partenaire qui porte pour sa part un marqueur.

3. Procédé selon la revendication 2, caractérisé en ce que l'on utilise comme partenaire un anticorps anti-haptène marqué.

4. Procédé selon l'une des revendications précédentes, caractérisé en ce que l'on utilise comme marqueur du nucléotide, de l'anticorps anti-haptène ou de l'autre partenaire du système de liaison un colorant fluorescent ou un colorant luminescent.

5. Procédé selon la revendication 2 ou 3, caractérisé en ce que l'on utilise comme marqueur de l'anticorps anti-haptène ou de l'autre partenaire du système de liaison un marqueur enzymatique et on réalise la détermination par le biais du clivage de substrat spécifique de l'enzyme de marquage.

6. Procédé selon l'une des revendications précédentes, caractérisé en ce que l'on maintient les cellules pendant l'introduction des liposomes et des nucléotides marqués dans un milieu de culture de cellules avec addition de sérum.

7. Procédé selon la revendication 4, caractérisé en ce que, lors de l'utilisation d'un colorant fluorescent comme marqueur, on réalise la détermination par le biais d'une immunofluorescence des cellules qui prolifèrent ou on mesure directement la fluorescence.

8. Procédé selon l'une des revendications précédentes, caractérisé en ce que, pour la détermination, on réalise un examen immunocytochimique des cellules qui prolifèrent.

9. Procédé selon l'une des revendications précédentes, caractérisé en ce que l'on réalise la détermination par le biais d'un ELISA cellulaire.

10. Procédé selon l'une des revendications précédentes, caractérisé en ce que l'on utilise des liposomes formés à partir de chlorure de N-[1-(2,3-dioléyloxy)propyl]-N,N,N-triméthylammonium.
